# EUROPÄISCHE PATENTSCHRIFT

(11) **EP 0 504 945 B1**
(45) Veröffentlichungstag und Bekanntmachung des Hinweises auf die Patenterteilung: **10.12.1997**
(21) Anmeldenummer: 92105007.6
(22) Anmeldetag: 23.03.1992
(51) Int. Cl.: A61B 5/08, A61B 5/0205, A61F 5/56, A61B 5/00, A61B 5/024, A61B 5/0456, A61B 7/00

(54) **Vorrichtung zur Diagnose und quantitativen Analyse von Apnoe und zur gleichzeitigen Feststellung anderer Erkrankungen**
Device for simultaneous diagnosis and quantitative analysis of apnea and other diseases
Dispositif pour la diagnose et l'analyse quantitative de l'apnée et la diagnose d'autres maladies

(30) Priorität: 22.03.1991 DE 4109529; 26.11.1991 DE 4138702
(43) Veröffentlichungstag der Anmeldung: 23.09.1992
(73) Patentinhaber: MAP Medizintechnik für Arzt und Patient GmbH, 82152 Martinsried (DE)
(72) Erfinder: Griebel, Peter, W-7800 Freiburg (DE)
(74) Vertreter: VOSSIUS & PARTNER

(56) Entgegenhaltungen:
- EP-A- 0 204 459
- EP-A- 0 357 249
- US-A- 4 802 485

## Beschreibung

Die Erfindung betrifft eine Vorrichtung zur ambulanten Erkennung und Diagnose des Schlaf-Apnoe-Syndroms, umfassend eine mobile Vorrichtung und einen Rechner. Dabei erfaßt man mit Hilfe der mobilen Vorrichtung die physiologischen Größen Herzfrequenz, Atmungslaute und Schnarchlaute des Patienten, speichert eine Vielzahl von jeweils für kurze Zeitintervalle erfaßten Sätzen der besagten Größen in kodierter Form in der mobilen Vorrichtung, überträgt sie in den Rechner und analysiert sie mit seiner Hilfe, wobei insbesondere zeitliche Schwankungen der einzelnen Größen und Korrelationen zwischen den verschiedenen Größen berücksichtigt werden.

Das Symptom der Schlaf-Apnoe ist gekennzeichnet durch das Zusammentreffen eines respiratorischen Versagens, einer erheblichen Hypoxämie (d.h. einer Herabsetzung des Sauerstoffgehalts im artiellen Blut) und von Herzrhythmusstörungen. Im Anschluß an einen Apnoe-Vorfall kommt es in der Regel zu einem heftigen Luftschnappen, oft auch zu einem aufschreckenden Erwachen (siehe z.B. M.J. Tobin, M.A. Cohn, M.A. Sackner: Breathing abnormalities during sleep, Arch. Intern. Med. 1983; Nr. 143, S. 1221 bis 1228).

Zunehmend wird die epidemologische Bedeutung der Schlaf-Apnoe erkannt; man weiß, daß lange und häufige Apnoe-Phasen im Schlaf oft mit kardiovaskulären und kardiopulmonalen Erkrankungen sowie mit tiefgreifenden psychophysischen Veränderungen einhergehen. Auswirkungen der Schlaf-Apnoe sind tagsüber eine exzessiv vermehrte Einschlafneigung (hier wird Schlaf-Apnoe statistisch als häufigste Ursache identifiziert) sowie das Auftreten von Ein- und Durchschlafstörungen (hier wird die Schlaf-Apone als fünfthäufigste Ursache beschrieben (R.M. Coleman, H.P. Roffwarg, S.J. Kennedy: Sleep-wake disorders based on polysomnographic diagnosis. A national cooperative study. JAMA 1982; Nr. 247, S. 997 bis 1003)).

Seit langem werden zur Untersuchung von Schlaf-Wach-Störungen Schlaflabors in Spezialkliniken genutzt, in denen eine Diagnose mittels polysomnigraphischer Auswertungen während des Schlafs vorgenommen werden kann. Diese Untersuchungen sind zeit- und kostenintensiv; sie können wegen der Vielzahl aufzunehmender Parameter nur bei einem stationären Aufenthalt des Patienten durchgeführt werden. Wegen des weit verbreiteten Auftretens der Schlaf-Apnoe muß eine äußerst große Anzahl von Patienten untersucht werden, was in stationären Schlaflabors kaum möglich ist. Daneben hat eine derartige stationäre Untersuchung den Nachteil, daß der Schlaf des Patienten durch die fremde Umgebung gestört wird. Dies verringert die Aussagekraft derartiger Untersuchungen. Daher gab es bereits eine Reihe von Bestrebungen, die Nachteile einer stationären Untersuchung zu beseitigen.

Eine Möglichkeit liegt darin, das Schlaf-Apnoe-Syndrom unter Vermeidung stationärer Untersuchungen in Schlaflabors ambulant mit Hilfe mobiler Erfassungs- und Speichervorrichtungen zu erkennen und zu diagnostizieren. So wird der europäischen Patentanmeldung 0 371 424 ein Verfahren und eine Vorrichtung zur ambulanten Erkennung und Diagnose des Schlaf-Apnoe-Syndroms entsprechend der eingangs erwähnten Art beschrieben. Dabei werden Herzpotentiale (d.h.EKG-Potentiale),mit zwei am Oberkörper eines Patienten anzubringenden Elektroden relativ zu einer dritten Elektrode gemessen und einem Spitzenwertdetektor zugeführt. Aus den zeitlichen Abständen zwischen den Spitzenwerten wird die Herzfrequenz bestimmt. Die Atmungs- und Schnarchlaute werden von einem am Kehlkopf des Patienten anzubringenden Elektret-Mikrofon aufgenommen und zwei verschiedenen Schwellenwertdetektoren zugeführt, wobei ein Schwellenwertdetektor über den gesamten Frequenzbereich (ungefähr 100 Hz bis 15 kHz) und der andere durch Dämpfung der hohen Frequenzen der Signale mit einem Filter nur in einem unteren, für Schnarchen typischen Frequenzbereich (ungefähr 100 Hz bis 800 Hz) sensitiv ist. Die Schwellenwertdetektoren sprechen an, wenn das anliegende Signal die eingestellten Schwellen übersteigt. Die Schwellen sind so eingestellt, daß mit dem erstgenannten Schwellenwertdetektor normales Atmen, mit dem Zweitgenannten Schnarchen nachgewiesen wird. Diese physiologischen Größen, nämlich Herzfrequenz und An- oder Abwesenheit von Atmen und Schnarchen, werden gemeinsam in Zeitintervallen von 1 s gemessen, und für jedes Zeitinterval binär kodiert in einem in der Vorrichtung befindlichen RAM-Speicher gespeichert. Die während einer Schlafperiode gespeicherten Daten werden aus der mobilen Vorrichtung in einen Rechner übertragen, und auf dem Rechner im Hinblick auf ein Schlaf-Apnoe-Syndrom analysiert. Aus den zeitlichen Veränderungen der Herzfrequenz, den Atmungs- und Schnarchlauten und den Korrelationen zwischen diesen Größen können Hinweise auf das Vorliegen einer Apnoe abgeleitet werden.

Eine weitere Vorrichtung zur ambulanten Überwachung von Schlaf-Apnoe ist in der US-A-4 802 485 offenbart.

Die Schwere einer Apnoe ist abhängig von der Häufigkeit der Vorfälle, von deren Tiefe und Dauer. Das beschriebene Verfahren mit der beschriebenen Vorrichtung erlaubt es zwar, das Vorliegen eines Apnoe-Vorfalls und dessen Dauer mit großer Empfindlichkeit nachzuweisen. Die Spezifität der Diagnose, die damit erreicht werden kann, ist jedoch unzureichend. Die Tiefe der Apnoe-Vorfälle ist nicht genau feststellbar. Information über die Körperlage des Patienten, die zur Diagnostik, etwa zur Unterscheidung zwischen aufrechter oder liegender Haltung des Patienten oder zur Abhängigkeit des Auftretens einer Obstruktion von der Körperlage wichtig ist, ist nicht vorhanden. Bei krankhaften Zuständen wie Polyneuropathie, starrem Altersherz oder Diabetes variiert die Herzfrequenz nicht, wie sonst bei Apnoe-Vorfällen, stark, sondern bleibt nahezu konstant. Bei Vorliegen derartiger Zustände kann eine Apnoe, in Ermangelung differentialdiagnostischer Möglichkeiten, wie etwa der Kenntnis der Sauerstoffsättigung des Bluts, nur schwierig diagnostiziert werden. In vielen Fällen bleibt daher bei Verwendung der im Stand der Technik bekannten Vorrichtung eine anschließende stationäre Untersuchung in einem Schlaflabor nötig.

Der Erfindung liegt daher die Aufgabe zugrunde, eine Vorrichtung zur ambulanten Erkennung und Diagnose des Schlaf-Apnoe-Syndroms zu schaffen, mit der eine so hohe Diagnosezuverlässigkeit erzielt werden kann, daß stationäre Nachuntersuchungen in einem Schlaflabor in der Regel nicht nötig sind. Die Vorrichtung sollte dazu neben der Feststellung der Häufigkeit und Dauer auch eine quantitative Bestimmung der Tiefe von Apnoe-Vorfällen und eine Erfassung der Körperlage des Patienten erlauben. Eine Apnoe-Diagnose sollte auch bei den oben genannten krankhaften Veränderungen möglich sein; umgekehrt sollten bei Unkenntnis ihres Vorhandenseins Hinweise auf diese Zustände gewonnen werden können.

Diese Aufgabe wird mit einer mobilen Vorrichtung der eingangs erwähnten Art gelöst, mit der man außerdem synchron zu den anderen erfaßten und gespeicherten Größen den Sauerstoffsättigungsgrad des Bluts und die Körperlage des Patienten erfaßt und kodiert speichert. Die gespeicherten Sätze dieser Größen können zusammen mit denen der anderen Größen in einen Rechner übertragen und mit seiner Hilfe analysiert werden, wobei man in der Analyse aus der Dauer und Tiefe von Perioden mit Sauerstoffentsättigung ein Maß für die Schwere einzelner Apnoe-Vorfälle gewinnt und man aus Änderungen der Körperlage ein Maß für die Apnoe-bedingte Schlafunruhe ableitet.

Vorteilhaft kann man in der Analyse mit Hilfe der Abhängigkeit des Auftretens von intermittierendem Schnarchen, zyklischen Variationen der Herzfrequenz und Sauerstoffentsättigung bei Apnoe-Vorfällen zwischen obstruktiver Apnoe, zentraler Apnoe und einigen anderen Schlafstörungen, wie Myoklonus, unterscheiden. Bei obstruktiver Apnoe treten die Vorfälle gehäuft in Rückenlage auf. Im allgemeinen führt ein Apnoe-Vorfall zu einer starken Variation der Herzfrequenz und zu einer Sauerstoff-Entsättigung des Bluts. Beobachtet man jedoch eine Entsättigung bei nahezu konstanter Herzfreqeunz, so kann man dies als einen Hinweis auf eine Polyneuropathie, ein starres Altersherz, Diabetes oder eine Herzinfarktgefahr auffassen. Zur Verbesserung der Qualität der Diagnose kennzeichnet man vorteilhaft besondere Vorkommnisse, die unerkannt in der Analyse stören könnten, wie etwa Hinlegen, Aufwachen oder Aufstehen, und quantifiziert die im Bett verbrachte Zeit. Dies kann dadurch geschehen, daß, zusätzlich zu der laufenden Registrierung der Körperposition, der Patient bei einem solchen Vorkommnis auf einen an der mobilen Vorrichtung befindlichen Druckschalter drücken kann. Aus der Messung des zeitlichen Verlaufs der Sauerstoffentsättigung des Bluts kann die Dauer und Tiefe eines Apnoe-Vorfalls bestimmt werden;
es kann daher auf die Bestimmung der Dauer aus den Atmungsgeräuschen verzichtet werden. Stattdessen können vorteilhaft nur die heftigen Schnarchgeräusche erfaßt werden, die beim Luftschnappen nach einem Apnoe-Vorfall auftreten.

Die erfindungsgemäße mobile Vorrichtung weist Mittel zur Erfassung der Herzfrequenz, der Atmungs- und Schnarchlaute, des Sauerstoffsättigungsgrads des Bluts und der Körperposition des Patienten in kurzen Zeitintervallen auf, Außerdem verfügt die Vorrichtung über Mittel zum Speichern einer Vielzahl von Sätzen dieser Signalgrößen in kodierter Form. Die Vorrichtung kann außerdem Mittel zur Kennzeichnung bestimmter Erfassungs-Zeitintervalle aufweisen, vorzugsweise handelt es sich hierbei um einen Druckschalter. Die Erfassungs-Zeitintervalle können für die verschiedenen aufzunehmenden und zu speichernden Größen verschiedenlang gewählt sein; bevorzugt sind Werte von 1 bis 10 s. Bei einer anderen bevorzugten Ausführungsform können auch kürzere Intervalle bis hinunter zu 0,1 s realisiert werden. Bei einer weiteren Ausführungsform ist die Länge der Erfassungs-Zeitintervalle variabel, und zwar kann die Länge von der mobilen Vorrichtung selbsttätig bedarfsgerecht eingestellt werden. Besonders kurze Zeitintervalle werden von der Vorrichtung dann gewählt, wenn ein besonders erfassungswürdiges Geschehen, also etwa ein möglicher Apnoe-Vorfall vorliegt.

Die Mittel zur Messung des Sauerstoffsättigungsgrads des Bluts umfassen mindestens eine Lichtquelle und mindestens einen Lichtempfänger, die an einer Extremität des Patienten befestigt werden und zur Messung der von der Extremität bewirkten Lichtabsorption oder Lichtreflexion dienen. Bei der (den) Lichtquelle(n) kann es sich z.B. um (eine) lichtemittierende Diode(n) und bei dem (den) Empfänger(n) um (einen) Fototransistor(en) handeln. Die Wellenlänge der verwendeten Lichtstrahlung wird vorzugsweise so gewählt, daß die mit einer Änderung des Sauerstoffgehalts des Bluts verbundene Farbänderung des Bluts optimal erkennbar ist; dies ist der Fall im Bereich des roten Lichts. Verwendet man Licht zweier verschiedener Wellenlängen, so wird die zweite verwendete Lichtstrahlung vorzugsweise so gewählt, daß ihre Reflexions- oder Transmissionseigenschaften vom Sauerstoffgehalt des Bluts möglichst unabhängig sind; dies ist der Fall im Bereich infraroten Lichts. Die Lichtstrahlung der zweiten Wellenlänge dient dazu, einen vom Sauerstoffgehalt unabhängigen Referenzwert zu gewinnen, mit dem das transmittierte bzw. reflektierte Licht der auf den Sauerstoffgehalt empfindlichen Lichtstrahlung normiert werden kann. Erfolgt die Messung der Farbänderung in Absorption, so sind Lichtquelle(n) und Lichtempfänger vorzugsweise gegenüberliegend an einem Befestigungselement angeordnet, wobei die lichtaussendende(n) und lichtempfangende(n) Seite(n) der Lichtquelle(n) bzw. des (der) Lichtempfänger einander zugewandt sind. Bei einer bevorzugten Ausführungsform handelt es sich bei dem Befestigungselement um eine Klemme, wobei Lichtquelle(n) und Lichtempfänger an gegenüberliegenden Bügeln der Klemme angeordnet sind. Bei einer anderen bevorzugten Ausführungsform handelt es sich bei dem Befestigungselement um eine flexible Schiene, die an einem Ende U-förmig gebogen ist, wobei Lichtquelle(n) und Lichtempfänger an den Schenkeln des "U" befestigt sind. Bei beiden Ausführungsformen wird das Befestigungselement so an der Extremität des Patienten befestigt, daß sich Körpergewebe zwischen der (den) Lichtquelle(n) und dem (den) Lichtempfänger(n) befindet.

Die Mittel zur Erfassung der räumlichen Lage des Patienten beinhalten vorzugsweise einen auf dem Oberkörper des Patienten zu befestigenden Hohlkörper mit einer darin befindlichen Kugel aus elektrisch leitendem Material. Vorteilhaft handelt es sich bei dem Hohlkörper um einen Hohltetraeder, an dessen Ecken durch die Kugel schließbare elektrische Kontakte angebracht sind. Der Hohltetraeder kann beispielsweise so orientiert sein, daß bei Rückenlage, Liegen auf der linken und rechten Seite und in aufrechter Haltung die Kugel jeweils in einer Ecke zu liegen kommt und dort den Kontakt schließt. Bei Bauchlage bleibt die Kugel auf einer Fläche des Tetraeders liegen und gibt keinen Kontakt.

Vorteilhaft werden die Mittel zur Erfassung der Atemgeräusche so eingerichtet, daß sie nur das heftige Schnarchgeräusch beim Luftschnappen nach einem Apnoe-Vorfall aufnehmen. Dies kann dadurch geschehen, daß die Schwelle des über den ganzen Frequenzbereich empfindlichen Schwellenwertdetektors hoch eingestellt wird.

Die zur Erfassung der Herzfrequenz, der Atmungs- und Schnarchlaute, des Sauerstoffsättigungsgrads des Bluts und der Körperposition des Patienten vorgesehenen Meßwertaufnehmer werden am Körper des Patienten befestigt und vorzugsweise mit der mobilen Vorrichtung über Signaldrähte verbunden. Bei einer anderen bevorzugten Ausführungsform werden die Signale dieser Meßwertaufnehmer drahtlos, z.B. durch elektromagnetische Wellen, an die mobile Vorrichtung übertragen. In diesem Fall entfällt die Notwendigkeit, die mobile Vorrichtung unmittelbar am Körper des Patienten zu befestigen. Statt eines an der mobilen Vorrichtung befindlichen Druckschalters zur Kennzeichnung besonderer Vorkommnisse kann man dann eine ebenfalls drahtlos ferngesteuerte Schaltvorrichtung verwenden.

Wie die vorstehenden Ausführungen zeigen, hat die Erfindung den Vorteil, daß durch die Messung des Sauerstoffsättigungsgrads eine Bestimmung der Schwere der Apnoe-Vorfälle und damit eine quantitativ genaue Analyse des Schlaf-Apnoe-Syndroms ermöglicht wird.

Die verschiedenen Kombinationen des Auftretens von Schnarchen und Änderungen der Herzfrequenz, der Sauerstoffsättigung und der Körperlage erlauben eine Bestimmung der Schlafunruhe und eine diagnostische Unterscheidung zwischen Apnoen, Hypopnoen, Myoklonus und anderen Schlafstörungen. Außerdem können Apnoe-Vorfälle auch bei krankhaften Zuständen, bei denen die Herzfrequenz nahezu konstant bleibt, diagnostiziert werden; auch ist die Entdeckung derartiger Zustände möglich. Insgesamt stellt die Erfindung somit gegenüber dem Stand der Technik wesentlich verbesserte und erweiterte Diagnosemöglichkeiten des Schlaf-Apnoe-Syndroms bereit. Es wurde festgestellt, daß die mit Hilfe der erfindungsgemäßen Vorrichtung gewonnenen Daten eine derartig hohe Signifikanz aufweisen, daß bei der überwiegenden Zahl von Probanden nach Auswertung der Daten durch Fachpersonal auf weiterführende Untersuchung verzichtet werden konnte. Trotz deren Einfachheit können somit mit der erfindungsgemäßen Vorrichtung Diagnosen erstellt werden, deren Zuverlässigkeit mit denen stationärer Langzeituntersuchungen in Schlaflabors vergleichbar ist, wobei jedoch die verfälschenden Einflüsse eines stationären Aufenthalts in einem Schlaflabor nicht vorhanden sind.

Anhand der nachfolgenden Figuren wird eine beispielhafte Ausführungsform der Erfindung näher beschrieben.

Hierbei zeigt:
- Figur 1: eine perspektivische Darstellung einer mobilen Erfassungs- und Speichervorrichtung;
- Figur 2: eine perspektivische Darstellung der Vorrichtung in Arbeitsposition am Körper eines Patienten;
- Figur 3: eine Seitenansicht eines Oxymeter-Sensors mit einer starren Fingerklemme zur Erfassung des Sauerstoffsättigungsgehalts mit Lichtabsorptionsmessung;
- Figur 4: eine Seitenansicht einer anderen Ausführungsform eines Oxymeter-Sensors entsprechend Fig. 3, jedoch mit einer flexiblen Schiene statt einer starren Klemme;
- Figur 5: eine Prinzipskizze der Erfassungs- und Speichervorrichtung;
- Figuren 6 und 7: graphische Darstellungen der erfaßten Größen bei verschiedenen Schlafstörungen, dargestellt als Funktion der Zeit.

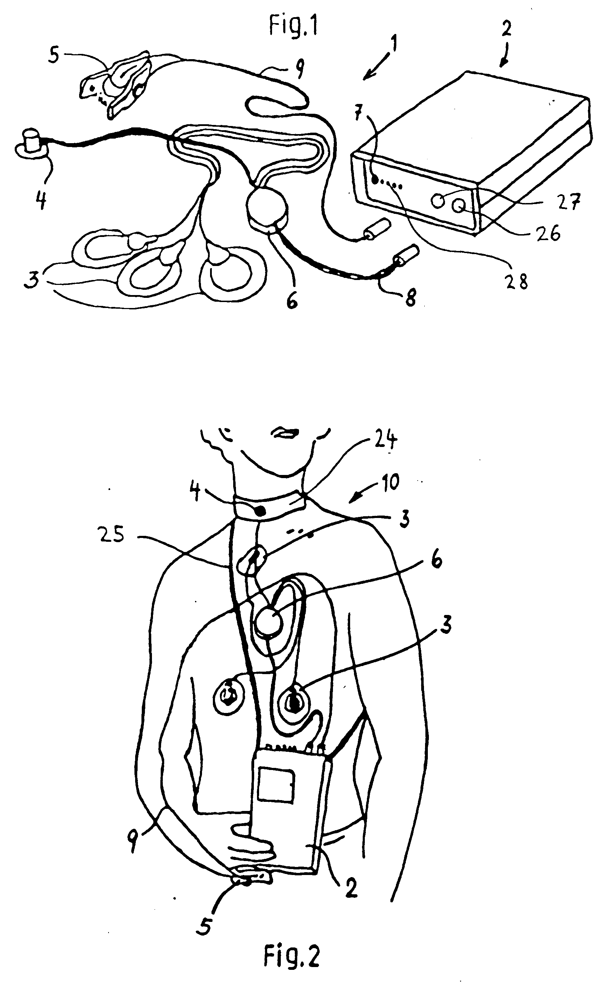

Die Erfassungs- und Speichervorrichtung 1 in Fig. 1 besteht aus dem eigentlichen Erfassung- und Speichergerät 2 mit einem frontseitig angeordneten Druckschalter 7 und folgenden verschiedenen Meßwertaufnehmern: drei EKG-Elektroden 3, einem Kehlkopfmikrofon 4, einem Oxymeter-Fingersensor 5, einem Lageaufnehmer 6 und zwei Verbindungskabeln 8, 9 mit je einem Stecker.

In Fig. 2 ist die Positionierung der Meßwertaufnehmer am Körper eines Patienten 10 gezeigt.

Die EKG-Elektroden 3 sind handelsübliche Einmalelektroden, die am Oberkörper des Patienten 10 befestigt werden; zwei von ihnen messen das Herzpotential (EKG-Potential) relativ zu der dritten Elektrode. Bei dem Kehlkopfmikrofon 4 handelt es sich um ein Elektretmikrofon, das mit einem ringförmigen Band 24 so am Hals des Patienten 10 befestigt wird, daß es am Kehlkopf anliegt. Die am Kehlkopf des Patienten 10 anliegende Seite des Mikrofons 4 ist mit einer ringförmigen isolierenden Polsterschicht und einem ringförmigen selbsthaftenden Einwegschutz versehen.

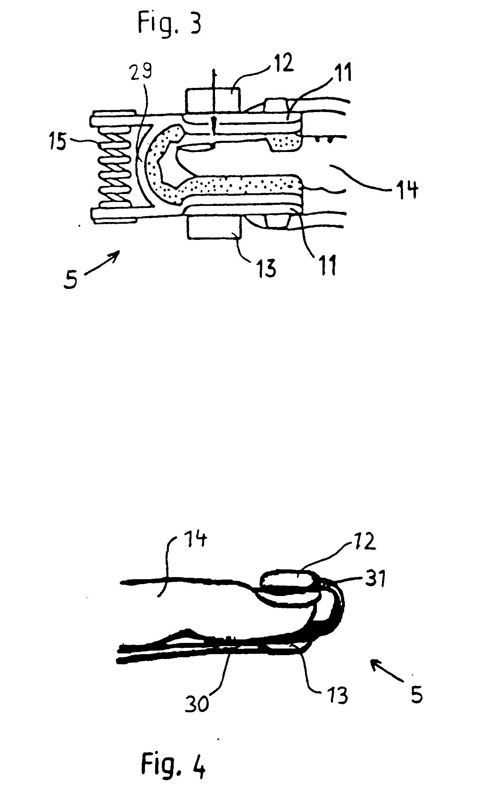

In Fig. 3 ist eine Ausführungsform eines Fingersensors 5 gezeigt. Er umfaßt zwei starre Klemmbügel 11, die durch einen elastischen Steg 29 miteinander verbunden sind. Auf einer Seite des Stegs 29 ist zwischen den Klemmbügeln 11 eine Druckfeder 15 angeordnet, durch die die Klemmbügel 11 auf der anderen Seite des Stegs zum Einklemmen eines Fingers 14 des Patienten 10 zueinander gedrückt werden. Auf dem oberen Klemmbügel 11 sind zwei lichtemittierende Dioden 12, und auf dem unteren zwei Fototransistoren 13 angeordnet. Ein Teil des von den lichtemittierenden Dioden 12 ausgesendeten Lichts durchquert den Finger 14 und wird von dem Fototransistor 13 empfangen, ein anderer Teil wird von dem Gewebe des Fingers absorbiert. Diese Absorptionsmessung erfolgt bei den Wellenlängen 660nm und 925 nm. Die Absorption des Lichts mit der ersten Wellenlänge hängt sehr stark vom Sauerstoffgehalt des Bluts ab; die Absorption des Lichts der zweiten Wellenlänge ist dagegen nahezu unabhängig hiervon.

Eine andere Ausführungsform eines Fingersensors 5 ist in Figur 4 gezeigt. Er umfaßt eine flexible Schiene 30, die an einem Ende U-förmig gebogen ist. Am Ende des freien Schenkels 31 des "U" sind zwei lichtemittierende Dioden 12 angeordnet. Auf dem anderen Schenkel befinden sich gegenüberliegend zwei Fototransistoren 13. Die U-förmige Schiene 30 wird von vorne über die Kuppe des Fingers 14 geschoben. Eine Fixierung des Fingersensors erfolgt mit Hilfe eines oder mehrere Bänder, die ringförmig um den Finger 14 und die U-Schiene 30 gelegt werden. Bei den Bändern kann es sich um Bänder mit Klettverschluß oder um Einmal-Klebebänder handeln. Bezüglich des verwendeten Lichts und dessen Absorptionseigenschaften gleicht diese Ausführungsform der Ausführungsform der Fig. 3.
Beide beschriebenen Ausführungsformen des Fingersensors 5 können von Laien einfach und auch richtig positioniert angelegt werden. Die Ausführungsform der Fig. 3 erlaubt ein besonders einfaches und schnelles Anlegen, während die Ausführungsform der Fig. 4 eine besonders sichere Fixierung und, da sie keinen Druck auf den Finger ausübt, einen hohen Tragekomfort vermittelt.
Der Lageaufnehmer wird in einer bestimmten Orientierung, die durch eine außen aufgedruckte Positionsbeschriftung vorgegeben ist, mit einem Klebering auf den Oberkörper des Patienten 10 befestigt. Der Lageaufnehmer 6 umfaßt einen Hohltetraeder mit einer darin befindlichen Kugel aus elektisch leitendem Material, in dessen Ecken durch die Kugel schließbare elektrische Kontakte angeordnet sind. Der Hohltetraeder ist so orientiert, daß bei Rückenlage, Liegen auf der linken Seite, der rechten Seite und in aufrechter Haltung die Kugel jeweils in einer Ecke zu liegen kommt und dort den Kontakt schließt; in Bauchlage bleibt die Kugel auf einer Fläche des Tetraeders liegen und gibt keinen Kontakt. Die aus dem Mikrofon, den EKG-Elektroden 3 und dem Lageaufnehmer 6 führenden Anschlußkabel werden zu einem einzigen Verbindungskabel 8 zusammengeführt, das mit Hilfe eines mehrpoligen Steckers mit dem Gerät 2 verbunden werden kann. Der Fingersensor 5 verfügt zur Verbindung mit dem Gerät 2 über ein eigenes Verbindungskabel 9 mit einem weiteren mehrpoligen Stecker.

Das Erfassungs- und Speichergerät 2 hat so kleine Abmessungen (190 x 135 x 45 mm) und ein so kleines Gewicht (ca. 700 g), daß es mit einem Schultergurt 25 unsichtbar unter der Kleidung auf dem Körper getragen werden kann. Das Gerät 2 weist auf seiner Frontseite zwei mehrpolige Buchsen 26, 27 auf,
in die die entsprechenden Stecker der Verbindungskabel 8, 9 gesteckt und gesichert werden können. Neben dem Druckschalter 7 sind auf dem Gerät 2 außerdem vier Leuchtdioden 28 zur Funktionskontrolle des Geräts 2 nach dem Anlegen angeordnet. Zur Übertragung der gespeicherten Daten an einen Rechner ist ein nicht-gezeigtes Daten-Übertragungskabel vorgesehen. Die Stromversorgung erfolgt wahlweise aus sechs Kleinbatterien mit 1,5 V oder entsprechenden Akkus.

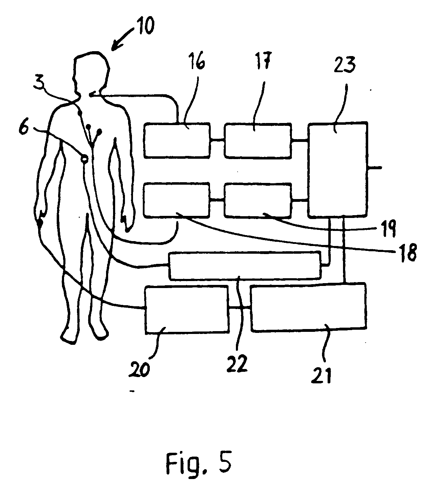

Die Prinzipskizze in Fig. 5 veranschaulicht die Signalverarbeitung in dem Erfassungs- und Speichergerät 2. Die vom Mikrofon 4 aufgenommenen Signale werden durch einen Verstärker 16 verstärkt und in zwei Kanäle verzweigt; die Signale eines Kanals werden anschließend durch ein Filter 17 geführt. Das Filter 17 dämpft Signale oberhalb 800 Hz mit einer Oktavdämpfung von 12 dB. Nicht gezeigt sind der ungefilterte Ast und die für beide Kanäle vorgesehenen Schwellenwertdetektoren. Der mit dem Filter 17 verbundene, im für Schnarchen typischen Frequenzbereich von ungefähr 100 Hz bis 800 Hz empfindliche Schwellenwertdetektor ist auf einen mittleren Schwellenwert eingestellt, derart, daß die Signale von normalen Schnarchlauten, nicht aber von leisen Atemgeräuschen die Schwelle überschreiten. Der zweite, auf dem ganzen Frequenzbereich von ungefähr 100 Hz bis 15 kHz ansprechende Schwellenwertdetektor ist auf einen so hohen Schwellenwert eingestellt, daß dieser im allgemeinen nur von Signalen überschritten wird, die von den sehr lauten Schnarch- und Luftschnappgeräuschen nach einem Apnoe-Vorfall herrühren. Die Information "Überschreiten bzw. Nichtüberschreiten der Schwellen" stellt bereits eine zur Speicherung und Weiterverareitung taugliche binare Größe dar.

Die von den Fototransistoren 13 des Fingersensors 5 herrührenden Signale werden zunächst von einem Signalverstärker 20 verstärkt. Anschließend wird in einem Entsättigungsanalysator 21 der Sauerstoffsättigungsgrad des Bluts ermittelt und binär kodiert. Die Signalhöhen entsprechen den Intensitäten der durch das Gewebe des Fingers 14 transmittierten, von den Fotodioden 12 emittierten Lichtwellenlängen. Da die Absorption des kurzwelligeren Lichts stark vom Sauerstoffgehalt des arteriellen Bluts abhängt, die des langwelligeren Lichts dagegen kaum vom Sauerstoffgehalt abhängt, läßt sich aus der Signalhöhe des kurzwelligeren Lichts nach Normierung auf die Signalhöhe des langwelligeren Lichts ein Maß für die Sauerstoffsättigung des Bluts ableiten. Dabei wird eine Genauigkeit von bis zu 2 % erreicht.

Die von den EKG-Elektroden 3 herrührenden Signale werden zunächst durch einen EKG-Verstärker 18 verstärkt und werden dann einem Herzfrequenzanalysator 19 zugeführt. Die dort gewonnenen Werte der Herzfrequenz werden binär kodiert.

Die vier möglichen, vom Lageaufnehmer 6 herrührenden Positionssignale werden in einem Lageanalysator 22 analysiert und ebenfalls binär kodiert. Nicht gezeigt in Fig. 4 ist der Druckschalter 7.

Alle genannten Größen werden laufend parallel aufgenommen, kodiert und nacheinander in Sätzen zeitlich zusammenge höriger Größen in einem RAM-Speicher 23 mit mindestens 128 kB Speicherkapazität gespeichert. Das Grund-Abtastintervall beträgt dabei 1 s; der Wert für die Sauerstoffsättigung wird alle 2 s, der für die Körperposition alle 10 s aufgenommen und erneuert. Die Aufzeichnungsdauer beträgt mindestens 22 h. Die gespeicherten Daten einer Aufzeichnungsperiode können über eine nicht-gezeigte Schnittstelleneinheit mit einer Übertragungsrate von 9200 Bauds über eine RS232-Schnittstelle eines nicht-gezeigten XT- oder AT-Personal-Computer übertragen werden. Durch Verwendung von Speicherbausteinen höherer Kapazität kann auch eine Gesamt-Speicherkapazität von mehreren Megabyte realisiert werden. Damit können kürzere Abtastintervalle oder eine längere Gesamt-Aufzeichnungsdauer eingestellt werden.

Bei Inbetriebnahme zu Beginn einer Aufzeichnungsperiode wird zunächst durch Anschluß des Verbindungskabels 8 an das Gerät 2 automatisch eine Funktionskontrolle ausgelöst. Sie dauert einige Minuten und wird mit Hilfe der Leuchtdioden 28 durchgeführt. Dabei ist jeweils eine der Leuchtdioden 28 für die Kontrolle der EKG-Funktion, der Sättigungsmessung und der beiden Atemgeräuschkanäle vorgesehen. Die dem EKG-Kanal zugeordnete Leuchtdiode wird mit dem Nachweis einer R-Zacke ausgelöst, und flackert so bei richtiger Funktion synchron mit den R-Zacken auf. Die dem Sauerstoffsättigungs-Kanal zugeordnete Leuchtdiode leuchtet während der ersten 30s der Funktionskontrolle auf, falls kein gültiger Wert vorhanden ist; dies ist z.B. der Fall, wenn der Fingersensor nicht in der richtigen Position am Finger befestigt ist. Bei ordnungsgemäßer Funktion bleibt diese Leuchtdiode erloschen. Die beiden den Atemgeräuschkanälen zugeordneten Leuchtdioden leuchten auf, wenn der jeweilige Schwellenwert überschritten ist. Bei ordnungsgemäßer Funktion sind diese beiden Leuchtdioden also im Normalzustand erloschen, und können durch simulierte Schnarchgeräusche bzw. sehr laute Atemgeräusche zum Aufleuchten gebracht werden. Nach dem Funktionstest beginnt das Gerät 2 mit der Aufnahme und Speicherung der genannten physiologischen Daten für die Dauer einer Datenaufnahmeperiode.

Nach Abschluß einer Datenaufnahmeperiode können die in dem mobilen Gerät 2 gespeicherten Daten zur Auswertung an einen Rechner übertragen werden. Bei der Auswertung können die Daten auf drei verschiedene Weisen bearbeitet und dargestellt werden:
i) Die gemessenen Größen (Atemgeräusche, Herzfrequenz, Sauerstoffsättigung, Körperposition) werden untereinander graphisch als Funktion der Zeit für die gesamte Erfassungsperiode dargestellt. Diese Darstellung zeigt quantitativ die gemeinsame zeitliche Entwicklung dieser Größe und damit insbesondere auch die Korrelationen zwischen ihnen. (Dabei kann eine komprimierte (z.B. 2h/Spur) oder eine vollständig expandierte Darstellung (z.B. 10 min/Spur) gewählt werden.) Sie hat den Vorteil, die gesamte aufgenommene Information zu enthalten; ihre Interpretation erfordert jedoch einen gewissen Zeitaufwand.
ii) Einzelne Meßgrößen werden zeitlich zusammengefaßt und in Form von Histogrammen und Tabellen dargestellt. Vorgesehen sind z.B. eine graphische Darstellung der Verteilungen der gemessenen Herzfrequenzwerte in aufeinanderfolgenden 10 min-Intervallen, eine graphische Darstellung der Verteilung der gemessenen Sauerstoffsättigungswerte, und Darstellungen der Entsättigungswerte als Funktion der Dauer und Häufigkeit ihres Auftretens in Form von Diagrammen und Tabellen. Diese Darstellungen machen nicht mehr die gesamte zeitliche Entwicklung und alle Korrelationen zwischen den gemessenen Größen deutlich, erlauben aber eine schnellere Interpretation.
iii) Aus den Daten wird die Zahl der Episoden, bei denen vermutlich ein Apnoe-Vorfall vorlag, direkt ermittelt und als Indexgröße mit der Einheit Episoden/Stunde angegeben. Es sind drei solcher Indexgrößen vorgesehen: Der Schnarchindex, der Herzfrequenzvariations-Index und der Sauerstoffentsättigungsindex. Es wurde festgestellt, daß wenigstens zwei übereinstimmende Indexgrößen einem "Apnoe-Index" äquivalent sind.

Zur Berechnung des Schnarchindex werden Schnarchpausen, die zwischen 11 und 60 s dauern, über den ganzen Erfassungszeitraum gezählt und durch die Anzahl der Stunden des Erfassungszeitraums dividiert. Schnarchpausen dieser Länge sind typisch für Apnoe-Vorfälle.

Zur Bestimmung des Herzfrequenzvariations-Index wird zunächst eine relative Herzfrequenz berechnet, indem man die momentane Herzfrequenz durch den laufenden Mittelwert der Herzfrequenz der vorangegangenen 300 s dividiert. Werte der relativen Herzfrequenz zwischen 90 % und 109 % werden der sogenannten 100%-Klasse zugeordnet; andere Werte liegen außerhalb dieser Klasse. Der Herzfrequenzvariations-Index gibt die Zahl der Ereignisse im Erfassungszeitraum an, bei denen die relative Herzfrequenz die 100%-Klasse verläßt und innerhalb von 11 bis 60 s wieder in die 100%-Klasse zurückkehrt, dividiert durch die Anzahl der Stunden des Erfassungszeitraums. Damit werden die Herzfrequenzvariationen erfaßt, wie sie synchron zum Apno-Geschehen auftreten.

Zur Bestimmung des Sauerstoffentsättigungs-Index wird zunächst der basale Sättigungswert ermittelt, indem die höchsten Sauerstoffsättigungswerte von 2 bis 10 vorangegangenen Messungen addiert werden und die Summe durch die Anzahl der Messungen dividiert wird. Eine Entsättigungsphase liegt vor, wenn der Sättigungswert um mindestens einen vorbestimmten Wert vom basalen Sättigungswert abfällt und dauert solange, bis im Wiederanstieg nach dem Abfall ein bestimmter Rücksättigungswert erreicht wird. Der Sauerstoffentsättigungs-Index gibt die Zahl der Entsättigungsphasen im Erfassungszeitraum an, dividiert durch die Anzahl der Stunden des Erfassungszeitraums. Er erfaßt die Entsättigungsphasen, wie sie ebenfalls synchron zum Apnoe-Geschehen auftreten.

Alle drei Indexgrößen geben direkt die Zahl von Vorfällen pro Stunde an. Bei einer obstuktiven Schlaf-Apnoe haben die drei Indexgrößen ungefähr denselben Wert. Große Abweichungen zwischen den drei Indexgrößen können Hinweise auf das Vorliegen besonderer krankhafter Veränderungen, wie eingangs erwähnt, geben. Insgesamt erlaubt die Angabe der drei Indexgrößen eine äußerst schnelle und signifikante Beurteilung des Apnoe-Geschehens.

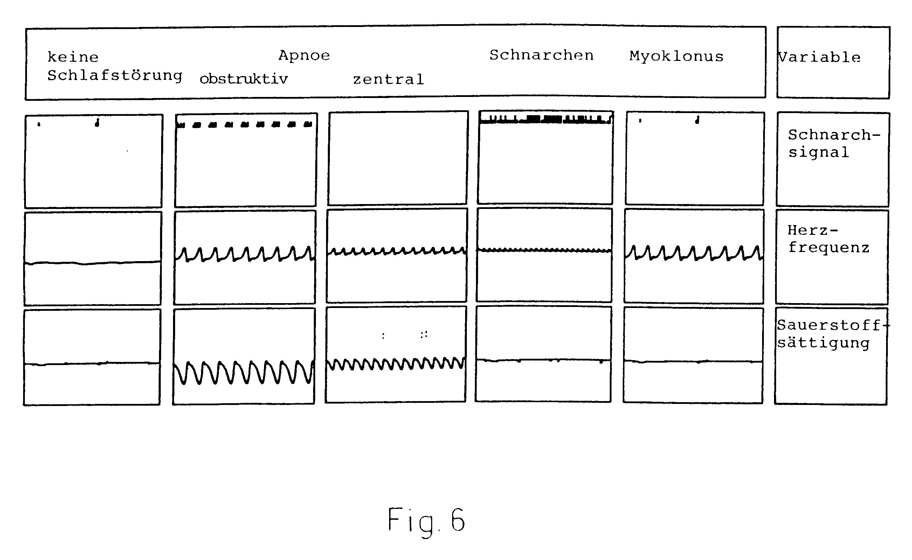

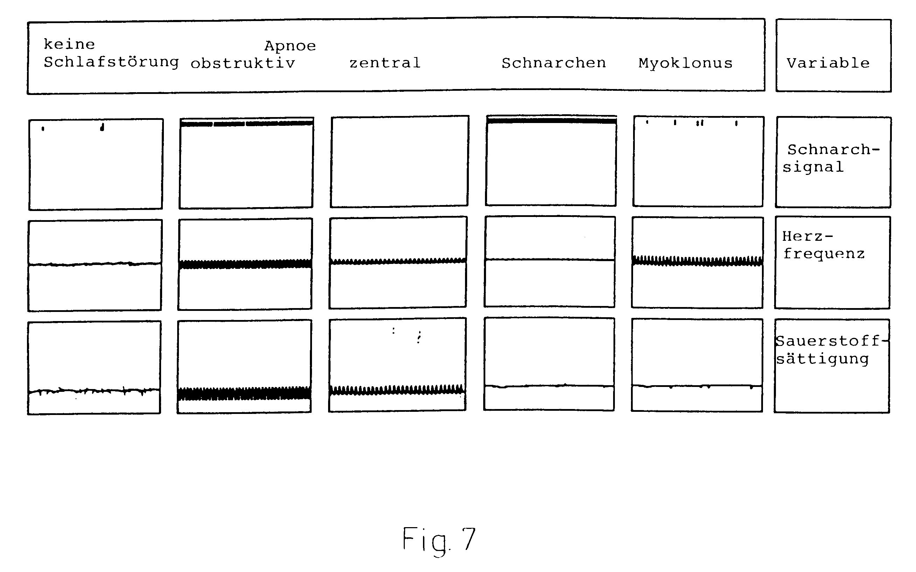

Die Figuren 6 und 7 zeigen Rohdaten-Darstellungen der Schnarch-, Herzfrequenz- und Sauerstoffsättigungs-Signale. In Figur 6 sind diese Signale in Intervallen von 10 Minuten, in Figur 7 von zwei Stunden aufgezeichnet. Gezeigt sind die Signale jeweils für verschiedene, mit Hilfe der Erfindung diagnostizierbare Schlafstörungen. Die gezeigten Kurven sind eine Darstellung der gespeicherten Rohdaten; sie können ausgehend von den gespeicherten Daten auf einfache Weise gezeichnet werden. Sie erlauben dem Arzt eine schnelle Überprüfung der verschiedenen Indexgrößen mit hoher Auflösung. Sie stellen auch eine einfach zu interpretierende Basis für die verschiedenen Diagnosen dar.

Eine tiefliegende Spur im Schnarchsignal steht für ein Signal mit niedriger Intensität. Eine hochliegende Spur steht für einen Geräuschpegel, der eine bestimmte Schwelle überschreitet. Änderungen der Körperlage werden ebenfalls dargestellt; zur Vereinfachung wurde dies jedoch in den Figuren weggelassen. Die Darstellung der Zwei-Stunden-Intervalle ist nützlich, um ein Gesamtbild zu erhalten. Damit können Intervalle identifiziert werden, in denen eine nähere Untersuchung angebracht ist.

Bei einem Gesunden ergeben die drei gezeichneten Variablen ein gleichförmiges Bild. Wiederholte Sauerstoffentsättigungen, Änderungen der Herzfrequenz und Schnarchen treten nicht auf. Ein Erwachen kann von einem plötzlichen Anstieg der Herzfrequenz und Änderungen der (nicht gezeigten) Körperlage abgeleitet werden.

Bei einem Patienten mit obstruktiver Schlaf-Apnoe (OSA) zeigen die drei gezeichneten Variablen ein zyklisches Muster. Bei einer OSA treten die folgenden drei Ereignisse immer gemeinsam auf: In Intervallen, in denen keine Atemgeräusche nachgewiesen werden, sinkt die Herzfrequenz und tritt eine Sauerstoffentsättigung auf. Eine Wiederaufnahme des Atmens ist gekennzeichnet durch explosives Schnarchen, zusammen mit einem Anstieg der Herzfrequenz und einer Sauerstoff-Rücksättigung. Es treten nur wenige Änderungen der Körperlage auf, bei der es sich typischerweise um eine Rückenlage handelt.

Die freien Intervalle zwischen den Schnarchsignal-Gruppen stellen die Apnoe-Intervalle dar. In manchen Fällen sind die Schnarchsignal-Gruppen, die bei der Wiederaufnahme des Atmens erscheinen, jedoch nicht durch völlig freie Zwischenräume getrennt. Vielmehr erscheint dort ein Muster von gleichmäßig beabstandeten schwarzen Schnarchsignal-Punkten. Dieses Muster repräsentiert eine obstruktive Hypoapnoe mit einer signifikanten Verengung der oberen Luftwege, und darf nicht mit kontinuierlichem Schnarchen verwechselt werden.

Bei einem Patienten mit zentraler Apnoe tritt typischerweise kein Schnarchen auf; auch spielt die Körperlage dann keine Rolle. Änderungen der Herzfrequenz und der Sauerstoffsättigung treten nach wie vor synchron auf, sind jedoch schwächer ausgeprägt.

Ein kontinuierliches Schnarchen ist gekennzeichnet durch das Auftreten kontinuierlicher Schnarchsignale ohne oder mit nur geringer Sauerstoffentsättigung und mit unregelmäßigen Variationen der Herzfrequenz. Dies deutet auf das Vorliegen eines Syndroms mit erhöhtem Widerstand der oberen Luftwege hin, das im Fall von Tagesmüdigkeit behandelt werden sollte.

Bei nächtlichen Myoklonien mit Erwachen beschränken sich die zyklischen Veränderungen auf die Herzfrequenz. Die Schnarch- und Sauerstoffsättigungs-Signale zeigen ein normales, von der Herzfrequenz unabhängiges Verhalten.

Falls Myoklonien zusammen mit obstruktiver Schlaf-Apnoe auftreten, zeigen sich auch das zyklische Schnarchen und die zyklischen Sauerstoffentsättigungen. Die Steigung des Anstiegs der Herzfrequenz ist bei Myoklonien jedoch größer als bei obstruktiver Schlaf-Apnoe.

Schlaflosigkeit macht sich durch eine große Zahl von Änderungen der Körperlage, viele unregelmäßige Variationen der Herzfrequenz und das Auftreten vieler unregelmäßiger Geräusche bemerkbar. Eine Sauerstoffentsättigung tritt dabei nicht auf.

Mit der erfindungsgemäßen Vorrichtung sind Diagnosen auch noch beim Vorliegen anderer Zustände möglich. Zum Beispiel zeigen Patienten mit Funktionsstörungen des autonomen Nervensystems nur sehr geringe Änderungen der Herzfrequenz, selbst wenn sie körperlich aktiv sind. Dasselbe trifft auf Personen mit Herzschrittmachern zu. Dennoch werden Patienten mit obstruktiver Schlafapnoe die wiederholten Sauerstoffentsättigungen und das intermittierende Schnarchen zeigen.

## Patentansprüche

1. Mobile Vorrichtung zur Erfassung und Speicherung physiologischer Größen eines Patienten (10) zur Diagnose des Schlaf-Apnoe-Syndroms mit
a) einer Einrichtung (4) zur Erfassung von Atmungslauten und
b) von Schnarchlauten,
c) einer Einrichtung (5) zur Erfassung und kodierten Speicherung des Sauerstoffsättigungsgrads des Bluts und mit
d) einer Einrichtung zum Speichern einer Vielzahl von jeweils für kurze Zeitintervalle erfaßten Sätzen der Größen a) bis c) in kodierter Form,
ferner gekennzeichnet durch
e) eine Einrichtung (3) zum Abtasten der Herzpotentiale und Erfassung der Herzfrequenz aufgrund der Herzpotentiale und
f) eine Einrichtung (6) zur Erfassung und kodierten Speicherung der Lageposition des Rumpfes des Patienten (10).

2. Mobile Vorrichtung nach Anspruch 1,
dadurch gekennzeichnet, daß
sie außerdem Mittel zur Kennzeichnung bestimmter Erfassungs-Zeitintervalle der Größen a) bis e) in kodierter Form aufweist.

3. Mobile Vorrichtung nach Anspruch 2,
dadurch gekennzeichnet, daß
die Mittel zur Kennzeichnung bestimmter Erfassungs-Zeitintervalle einen Druckschalter (7) beinhalten.

4. Mobile Vorrichtung nach einem der Ansprüche 1 bis 3,
dadurch gekennzeichnet, daß
die Einrichtung zur Erfassung des Sauerstoffsättigungsgrads des Bluts einen Sensor mit mindestens einer Lichtquelle (12) und mindestens einem Lichtempfänger (13) zur Messung der von einer Extremität (14) des Patienten bewirkten Lichtabsorption oder -reflexion umfasst.

5. Mobile Vorrichtung nach Anspruch 4,
dadurch gekennzeichnet, daß
die Lichtquelle (12) und der (die) Lichtempfänger (13) gegenüberliegend an einem Befestigungselement angeordnet sind, wobei die lichtaussendende und lichtempfangende Seite der Lichtquelle (12) bzw. des Lichtempfängers (13) einander zugewandt sind.

6. Mobile Vorrichtung nach Anspruch 5,
dadurch gekennzeichnet, daß
das Befestigungselement von einer Klemme (5) gebildet wird, wobei Lichtquelle(n) (12) und Lichtempfänger (13) an gegenüberliegenden Bügeln (11) der Klemme (5) angeordnet sind.

7. Mobile Vorrichtung nach Anspruch 5,
dadurch gekennzeichnet, daß
das Befestigungselement von einer flexiblen Schiene (30) gebildet wird, die an einem Ende U-förmig gebogen ist, wobei Lichtquelle(n) (12) und Lichtempfänger (13) an den Schenkeln des "U" angeordnet sind.

8. Mobile Vorrichtung nach einem der Ansprüche 1 bis 7,
dadurch gekennzeichnet, daß
die Einrichtung zur Erfassung der Lageposition des Rumpfes des Patienten einen auf dem Oberkörper des Patienten (10) zu befestigenden Hohlkörper (6) mit einer darin befindlichen Kugel aus elektrisch leitendem Material beinhaltet.

9. Mobile Vorrichtung nach Anspruch 8,
dadurch gekennzeichnet, daß
es sich bei dem Hohlkörper (6) um einen Hohltetraeder handelt, in dessen Ecken durch die Kugel schließbare elektrische Kontakte angeordnet sind.

10. Mobile Vorrichtung nach einem der Ansprüche 1 bis 9,
dadurch gekennzeichnet, daß
die Einrichtung zur Erfassung der Atmungs- und Snarchlauten so eingerichtet ist, daß sie nur das heftige Schnarchgeräusch beim Luftschnappen nach einem Apnoe-Vorfall aufnimmt.

## Claims

1. Mobile apparatus for detecting and storing physiological parameters in a patient for use in diagnosing the sleep apnea syndrome comprising
a) means (4) for sensing respiratory sounds and
b) snoring sounds,
c) means (5) for sensing and storing in coded form the degree of oxygen saturation of the blood and
d) means for storing a plurality of sets of parameters a) to c) obtained for short time intervals, in coded form,
characterized in that the mobile apparatus further comprises
e) means (3) for detecting heart potentials and sensing the heart rate based on the heart potentials and
f) means (6) for sensing and coded storage of the bodily position of the patient (10).

2. The mobile apparatus according to claim 1, characterized in that it furthermore comprises means for the identification in coded form of certain time intervals during which parameters a) to e) are detected.

3. The mobile apparatus according to claim 2, characterized in that the means for the identification of certain time intervals include a push-button switch (7).

4. The mobile apparatus according to any one of claims 1 to 3, characterized in that the means for sensing the degree of oxygen saturation of the blood include a sensor with at least one light source (12) and at least one light receiver (13) for measuring light absorption or reflection produced by an extremity (14) of the patient.

5. The mobile apparatus according to claim 4, characterized in that the light source (12) and the light receiver(s) (13) are oppositely disposed on a fastening means, the light emitting and light receiving side of the light source (12) and light receiver (13), respectively, facing one another.

6. The mobile apparatus according to claim 5, characterized in that the fastening means is formed by a clip (5), said light source(s) (12) and light receiver(s) (13) being disposed on opposite legs (11) of the clip (5).

7. The mobile apparatus according to claim 5, characterized in that the fastening element is formed by a flexible strip (30) which is bent U-wise at one end, said light source(s) (12) and light receiver(s) (13) being disposed on limbs of the "U".

8. The mobile apparatus according to any one of claims 1 to 7, characterized in that the means for detecting the bodily position of the patient include a hollow body (6) to be fastened to the upper body of the patient (10) with a ball of electrically conductive material situated therein.

9. The mobile apparatus according to claim 8, characterized in that the hollow body (6) is a hollow tetrahedron in whose corners electrical contacts are disposed which can be closed by the ball.

10. The mobile apparatus according to any one of claims 1 to 9, characterized in that the means for the detection of the respiratory and snoring sounds are arranged such that they pick up only loud, snoring sounds resulting from gasping for air after an episode of apnea.

## Revendications

1. Dispositif mobile pour la saisie et la mémorisation de valeurs physiologiques d'un patient (10) pour le diagnostic du syndrome de l'apnée de sommeil avec
a) un appareil (4) pour la saisie des bruits respiratoires et
b) des bruits de ronflement,
c) un appareil (5) pour la saisie et la mémorisation codée du taux de saturation en oxygène du sang et
d) un dispositif pour la mise en mémoire d'une pluralité d'ensembles de valeurs saisies chaque fois pour de courts intervalles de temps pour les valeurs a) jusqu'à c) sous une forme codée,
caractérisé de plus par
e) un appareil (3) pour la détection des potentiels cardiaques et la saisie de la fréquence cardiaque sur la base des potentiels cardiaques et
f) un appareil (6) pour la saisie et la mémorisation codée de la position allongée du tronc du patient (10).

2. Dispositif mobile selon la revendication 1, caractérisé en ce qu'
il présente en outre des moyens pour la caractérisation d'intervalles de temps de saisie déterminés des grandeurs a) jusqu'à e) sous forme codée.

3. Dispositif mobile selon la revendication 2, caractérisé en ce que
les moyens destinés à la caractérisation d'intervalles de temps de saisie déterminés contiennent un interrupteur à pression (7).

4. Dispositif mobile selon l'une des revendications 1 à 3,
caractérisé en ce que
l'appareil destiné à la saisie du taux de saturation en oxygène du sang comporte un capteur avec au moins une source de lumière (12) et au moins un récepteur de lumière (13) pour mesurer la réflexion ou l'absorption lumineuse exercée par une extrémité (14) du patient.

5. Dispositif mobile selon la revendication 4,
caractérisé en ce que
la source de lumière (12) et le (les) récepteur(s) de lumière (13) sont disposés en face d'un élément de fixation de sorte que le côté récepteur et le côté émetteur de lumière de la source de lumière (12) ou du récepteur de lumière (13) sont dirigés l'un vers l'autre.

6. Dispositif mobile selon la revendication 5,
caractérisé en ce que
l'élément de fixation est constitué par une pince (5), la ou les source(s) de lumière (12) et le ou les récepteur(s) de lumière (13) sont disposés sur des étriers opposés (11) de la pince (5).

7. Dispositif mobile selon la revendication 5,
caractérisé en ce que
l'élément de fixation est formé par une glissière souple (30) qui est cintrée en forme de U sur une extrémité, la ou les source(s) de lumière (12) et le ou les récepteur(s) de lumière (13) étant disposés sur les branches du 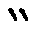 U*"*.

8. Dispositif mobile selon l'une des revendications 1 à 7,
caractérisé en ce que
l'appareil destiné à la saisie de la position allongée du tronc du patient contient un corps creux (6) à fixer sur la partie supérieure du corps du patient (10) avec une sphère à l'intérieur de ce corps creux en un matériau conducteur électrique.

9. Dispositif mobile selon la revendication 8,
caractérisé en ce que
le corps creux (6) est un tétraèdre creux dans les angles duquel sont disposés des contacts électriques pouvant être fermés par la sphère.

10. Dispositif mobile selon l'une des revendications 1 à 9,
caractérisé en ce que
l'appareil destiné à la saisie des bruits respiratoires et de ronflement est disposé de telle sorte qu'il ne perçoit que les bruits de ronflement violents lors de crises de suffocation ou halètement après un incident d'apnée.
